# EUROPEAN PATENT APPLICATION

(11) **EP 4 834 219 A1**
(43) Date of publication of application: **04.11.2026**
(21) Application number: 26174903.0
(22) Date of filing: 27.04.2026
(51) Int. Cl.: A61B 5/145

(54) **PHYSIOLOGICAL INFORMATION MONITORING DEVICE**

(30) Priority: 28.04.2025 CN 202510551224
(71) Applicant: Shanghai United Imaging Microelectronics Technology Co., Ltd., Shanghai 201899 (CN)
(72) Inventor: LIU, Wei, Shanghai, 201899 (CN)
(74) Representative: Balder IP Law, S.L.

(57) **Abstract**

The present application relates to a physiological information monitoring device, including a base, a transmitter assembly, a detecting member, and a power-supplying member. The transmitter assembly at least includes a transmitter. The transmitter assembly is disposed on the base. The power-supplying member and the detecting member are disposed in the base. The detecting member has a detecting terminal, and the detecting terminal extends through and protrudes from the base. The base is electrically connected to the transmitter and the detecting member, respectively. The power-supplying member is electrically connected to the transmitter. The transmitter assembly defines an auxiliary mounting hole extending through a thickness thereof. When the transmitter assembly is mounted on the base, the auxiliary mounting hole faces the detecting member, and the auxiliary mounting hole allows the auxiliary device to extend therethrough, such that the auxiliary device is connected to the detecting member.

## Description

### TECHNICAL FIELD

The present application relates to the field of physiological information monitoring technology, and in particular to a physiological information monitoring device.

### BACKGROUND

A physiological information monitoring device is a monitoring device capable of comfortably, conveniently, and continuously monitoring the concentration of a biological analyte in the human body, and converting the concentration value into a measurable parameter such as an electrical signal.

The physiological information monitoring device typically includes a sensor and a transmitter. The sensor is configured to detect the analyte concentration, while the transmitter is configured to send the detection result signal to a terminal device. The sensor and the transmitter are electrically connected for signal transmission. In related art, when in use, the monitoring device is usually deployed with an auxiliary device. The detection probe of the sensor is implanted subcutaneously into the patient via the auxiliary device.

However, in related art, the assembly between the monitoring device and the auxiliary device is relatively complicated, and the stability of the electrical connection between the sensor and the transmitter is relatively low.

### SUMMARY

An embodiment of the present application provides a physiological information monitoring device, which does not require removal of the transmitter when mounting or removing the auxiliary device, thereby simplifying the assembly between the monitoring device and the auxiliary device, and avoiding the issue that the removal of the transmitter can easily lead to poor assembly stability between the base and the transmitter, thus maximally ensuring the stability of the electrical connection between the base and the transmitter.

An embodiment of the present application provides a physiological information monitoring device configured to cooperate with an auxiliary device. The physiological information monitoring device includes a base, a transmitter assembly, a detecting member, and a power-supplying member. The transmitter assembly at least includes a transmitter.

The transmitter assembly is disposed on the base. The power-supplying member and the detecting member are disposed in the base. The detecting member has a detecting terminal. The detecting terminal extends through and protrudes from the base. The base is respectively electrically connected to the transmitter and the detecting member. The power-supplying member is electrically connected to the transmitter.

The transmitter assembly defines an auxiliary mounting hole extending through the thickness of the transmitter assembly. When the transmitter assembly is mounted on the base, the auxiliary mounting hole faces the detecting member, and the auxiliary mounting hole allows the auxiliary device to extend therethrough, such that the auxiliary device is connected to the detecting member.

In an embodiment, the base includes a base seat. The base seat has an open structure at a side adjacent to the transmitter assembly. The base seat defines a through hole, and the detecting terminal extends through the through hole.

The auxiliary mounting hole and the through hole are coaxial and in communication with each other.

In an embodiment, the transmitter assembly includes a transmitter. The transmitter is disposed on the base and covers at least part of the open structure. The auxiliary mounting hole is located in the transmitter.

In an embodiment, the transmitter assembly includes a transmitter and a covering member. The covering member is disposed on the base and covers at least part of the open structure.

The transmitter is disposed on a side of the covering member away from the power-supplying member. The auxiliary mounting hole is located in the covering member. The transmitter is positioned to avoid the auxiliary mounting hole.

In an embodiment, the covering member includes a covering portion and a connecting portion. The covering portion covers at least part of the open structure.

The connecting portion and the transmitter are located on the same side of the covering portion. The transmitter horizontally abuts against the connecting portion. The auxiliary mounting hole extends through both the connecting portion and the covering portion. The transmitter is configured to extend through the covering portion and is electrically connected to the base.

In an embodiment, at a position where the connecting portion abuts against the transmitter, one of the connecting portion and the transmitter includes a snap buckle, another one of the connecting portion and the transmitter includes a snap groove, and the snap buckle is snap-fitted into the snap groove.

Alternatively, an end of the connecting portion is rotatably connected to an end of the transmitter via a rotating shaft, and another end of the transmitter is rotatable around the rotating shaft relative to the connecting portion.

In an embodiment, at a position where the connecting portion abuts against the transmitter, one of the connecting portion and the transmitter has a convex surface, and the other of the connecting portion and the transmitter has a concave surface.

In an embodiment, one of the base seat and the transmitter has an inserting protrusion, the other of the base seat and the transmitter has a fixing buckle, and the inserting protrusion is assembled into the fixing buckle.

In an embodiment, the base seat is provided with a first conductive member. The transmitter includes a housing, a circuit board, and a second conductive member. The circuit board and the second conductive member are disposed in the housing. The second conductive member protrudes from a side of the housing facing the base. The first conductive member is respectively electrically connected to the detecting member and the second conductive member. The second conductive member is electrically connected to the circuit board.

In an embodiment, the base seat includes a mounting structure defining a mounting groove, and the first conductive member is disposed in the mounting groove. The mounting groove structure defines a mounting notch, and at least a part of the detecting member extends into the mounting groove via the mounting notch.

In an embodiment, the base seat includes a limiting structure defining a limiting groove, the limiting groove surrounds the opening of the through hole. A portion of the detecting member is surrounded by the limiting groove.

The limiting structure defines a limiting notch, the limiting notch is opposite to the mounting notch. At least part of the detecting member extends from the mounting notch to the mounting groove.

In an embodiment, the power-supplying member includes a third conductive member. The transmitter includes a fourth conductive member. The fourth conductive member is disposed in the housing and protrudes from the side of the housing facing the base. The third conductive member is electrically connected to the fourth conductive member.

The third conductive member is located on a side adjacent to the mounting groove.

In an embodiment, the power-supplying member includes a battery and an electrode plate connected to the battery. The electrode plate is electrically connected to the third conductive member.

In an embodiment, the power-supplying member further includes a substrate, the substrate is located in the base, and the battery and the third conductive member are both disposed on the substrate.

In an embodiment, the covering member defines an opening. When the covering member is mounted on the base, the first conductive member and the third conductive member are exposed through the opening.

In an embodiment, the covering member includes a first sealing member, which is disposed around the periphery of the opening, and/or the transmitter includes a second sealing member, which is disposed on the side adjacent to the base, the second sealing member surrounds the second conductive member and the fourth conductive member.

In an embodiment, one of the first conductive member and the second conductive member is a rigid member, and the other of the first conductive member and the second conductive member is a flexible member; and/or

one of the third conductive member and the fourth conductive member is a rigid member, and the other of the third conductive member and the fourth conductive member is a flexible member.

The physiological information monitoring device provided in the embodiments of the present application includes a base and a transmitter assembly, wherein the transmitter assembly defines an auxiliary mounting hole. In this way, after the base is assembled with the transmitter, the auxiliary device can be directly inserted into the base through the auxiliary mounting hole and connected to the detecting member in the base. Thus, there is no need to mount the auxiliary device onto the base before mounting the transmitter onto the base. During disassembly, there is no need to remove the transmitter before detaching the auxiliary device from the base. Therefore, the present application simplifies the assembly between the monitoring device and the auxiliary device, saves assembly time, thereby saving time for monitoring while ensuring the assembly stability between the base and the transmitter. Moreover, the interference with the electrical connection between the base and the transmitter caused by the mounting or removal of the auxiliary device is reduced, thereby maximally guaranteeing the stability of the electrical connection between the base and the transmitter.

### BRIEF DESCRIPTION OF THE DRAWINGS

In order to illustrate the embodiments or examples of the present application more clearly, the drawings used in the description of the embodiments or examples will be described briefly. Apparently, the following described drawings are merely for some embodiments of the present application, and other drawings can be derived based on the following drawings by those of ordinary skill in the art without any creative effort.
FIG. 1 is a schematic structural view of a physiological information monitoring device provided in an embodiment of the present application.
FIG. 2 is a schematic exploded view of a physiological information monitoring device provided in an embodiment of the present application.
FIG. 3 is a schematic view of a base assembled with a power-supplying member, in a physiological information monitoring device provided in an embodiment of the present application.
FIG. 4 is a schematic view of a covering member assembled with a base, in a physiological information monitoring device provided in an embodiment of the present application.
FIG. 5 is a schematic structural view of a transmitter of a physiological information monitoring device provided in an embodiment of the present application.
FIG. 6 is a first view illustrating an assembly process of a transmitter with a covering member, in a physiological information monitoring device provided in an embodiment of the present application.
FIG. 7 is a second view illustrating an assembly process of the transmitter with the covering member, in the physiological information monitoring device provided in the embodiment of the present application.
FIG. 8 is a schematic view illustrating a completed assembly of the transmitter with the covering member, in the physiological information monitoring device provided in the embodiment of the present application.
FIG. 9 is a cross-sectional view of a base assembled with a transmitter, provided in an embodiment of the present application.

Reference signs:
100: base, 110: base seat, 120: through hole, 130: fixing buckle, 140: first conductive member, 150: mounting groove, 151: mounting notch, 160: limiting groove, 161: limiting notch, 170: auxiliary assembly groove, 180: recessed structure, 200: transmitter assembly, 210: transmitter, 211: snap buckle, 212: concave surface, 213: inserting protrusion, 214: second conductive member,215: fourth conductive member, 216: housing, 217: second sealing member, 220: covering member, 221: covering portion, 222: connecting portion, 223: snap groove, 224: convex surface, 225: opening, 226: first sealing member, 230: auxiliary mounting hole, 300: detecting member, 310: detecting terminal, 400: power-supplying member, 410: battery, 420: electrode plate, 430: third conductive member, 440: substrate.

### DETAILED DESCRIPTION

The embodiments of the present application will be described in detail with reference to the accompanying drawings in order to make the objectives, features, and advantages of the present application more apparent and understandable. Many specific details are disclosed in the following description to facilitate a comprehensive understanding of the present application. However, the present application can be implemented in various ways different from those described herein, and those skilled in the art may make similar improvements without departing from the concept of the present application. Therefore, the present application is not limited to the specific embodiments disclosed below.

In the description of the present application, it should be understood that, if the terms "central", "longitudinal", "transverse", "length", "width", "thickness", "upper", "lower", "front", "back", "left", "right", "vertical", "horizontal", "top", "bottom", "inner", "outer", "clockwise", "counterclockwise", "axial", "radial", "circumferential", etc. appear, the orientation or position relationships indicated by these terms are based on the orientation or position relationships shown in the accompanying drawings and are intended to facilitate the description of the present application and simplify the description only, rather than indicating or implying that the apparatus or element referred to must have a particular orientation or be constructed and operated in a particular orientation, and therefore are not to be interpreted as limiting the present application.

In addition, if the terms "first" and "second" appear, these terms are used for descriptive purposes only, and cannot be construed as indicating or implying a relative importance, or implicitly specifying the number of the indicated technical features. Thus, the quantity of the feature defined with "first" or "second" may explicitly or implicitly be at least one. In the description of the present application, if the term "a plurality of" appears, it means at least two, such as two, three, unless otherwise defined explicitly and specifically.

It should be noted that an element, when being referred to as being "fixed" or "disposed" on another element, may be directly fixed or disposed onto the other element or via an intermediate element. An element, when being referred to as being "connected to" another element, may be directly connected to another element or connected to another element via an intermediate element. If present, such terms as "vertical", "horizontal", "up", "down", "left", "right" and the like used herein are for illustrative purposes only and are not meant to be the only ways for implementing the present application.

In related art, when in use, the monitoring device is usually deployed with an auxiliary device. The detection probe of the sensor is implanted subcutaneously into the patient via the auxiliary device. Specifically, since the existing transmitter is of an integral structure without any assembly holes, when mounting the auxiliary device, the auxiliary device needs to be mounted on the sensor first, so that the auxiliary device is connected to the detection probe. The detection probe is then implanted subcutaneously into the patient under the action of the auxiliary device. After that, the transmitter is connected to the sensor, at which time the monitoring circuit is closed and the monitoring begins. During the monitoring, the transmitter transmits data signals to the terminal device in real time. When detaching or replacing the auxiliary device, the transmitter needs to be removed first, and then the auxiliary device is detached from the sensor.

However, the existing assembly approach is relatively complicated. Additionally, during the mounting or removal of the auxiliary device, the transmitter needs to be removed and mounted correspondingly, resulting in poor assembly stability and thus lowering the electrical connection stability between the transmitter and the sensor.

To address the above issues, embodiments of the present application provide a physiological information monitoring device that simplifies the assembly approach between the monitoring device and the auxiliary device, saves assembly time, thereby saving time for monitoring while ensuring the assembly stability between the base and the transmitter. Moreover, the interference with the electrical connection between the base and the transmitter caused by the mounting or removal of the auxiliary device is reduced, thereby maximally guaranteeing the stability of the electrical connection between the base and the transmitter.

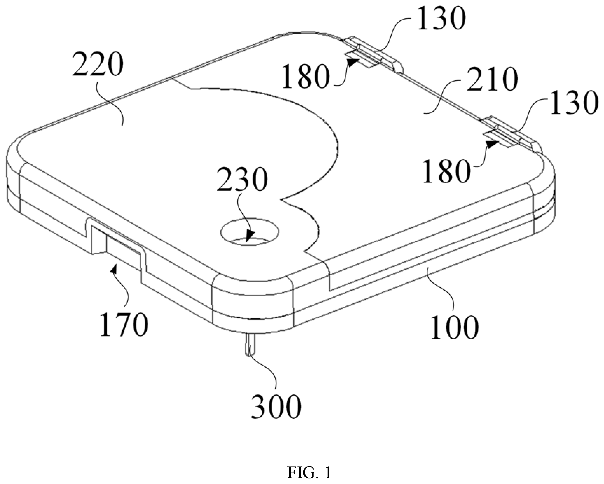

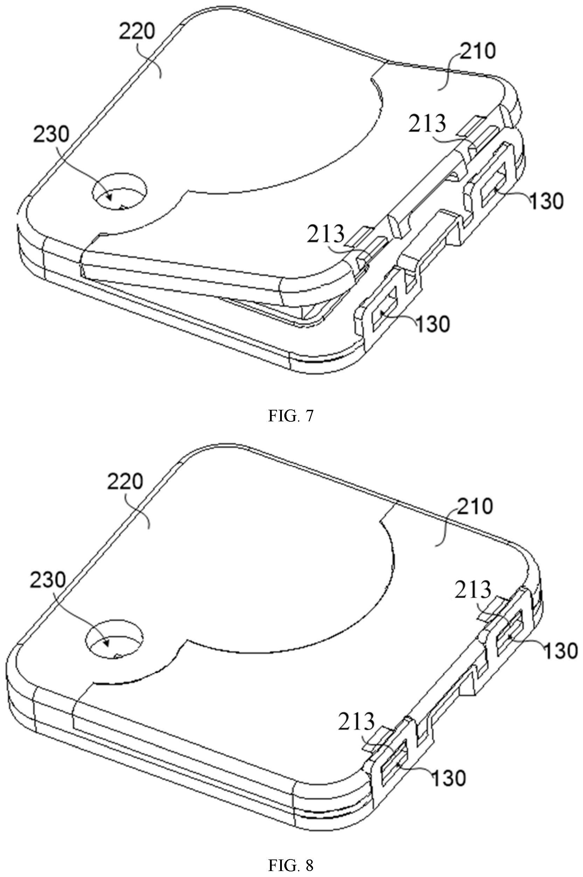

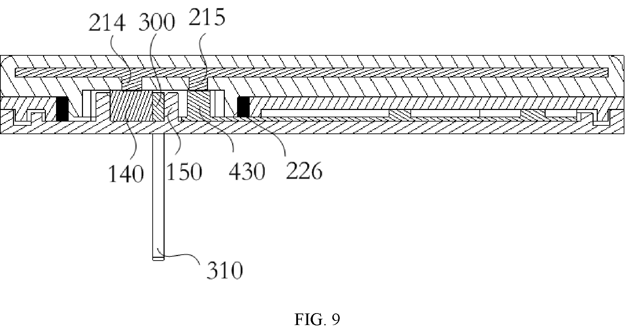

The specific structure of the physiological information monitoring device provided in the embodiments of the present application will be described below in conjunction with FIG. 1 to FIG. 9.

An embodiment of the present application provides a physiological information monitoring device configured to cooperate with an auxiliary device. For example, the physiological information monitoring device can be configured to monitor a physiological parameter such as blood sugar levels, heart rates, blood pressures, body temperatures, etc., which is not limited herein.

In an embodiment, the physiological information monitoring device is configured to continuously monitor blood glucose levels and its sensor is wearable on the human body, which is taken as an example for description.

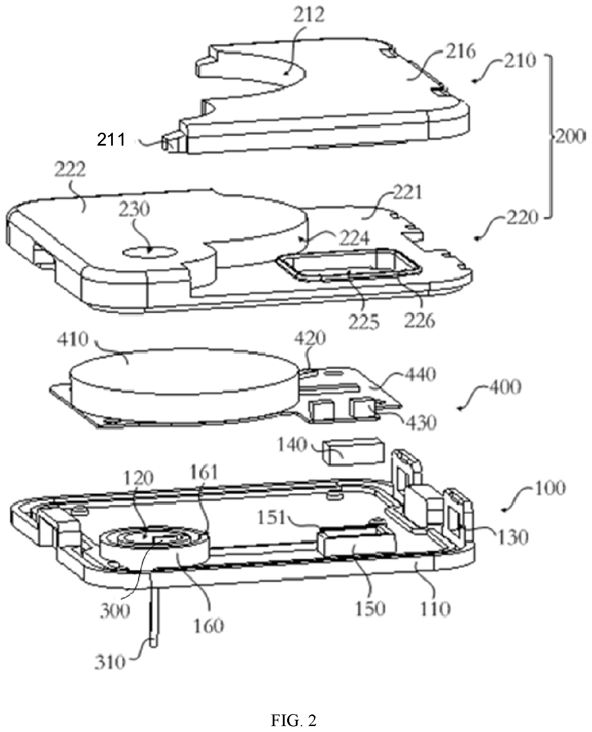

In an embodiment, as shown in FIG. 1 and FIG. 2, the physiological information monitoring device includes a base 100, a transmitter assembly 200, a detecting member 300, and a power-supplying member 400. The transmitter assembly 200 at least includes a transmitter 210.

In the present embodiment, the transmitter assembly 200 is disposed on the base 100, the power-supplying member 400 and the detecting member 300 are disposed in, i.e., enclosed with, the base 100, and the detecting member 300 has a detecting terminal 310. The detecting terminal 310 extends through and protrudes from the base 100, and is configured to be implanted subcutaneously into the human body. The base 100 is respectively electrically connected to the transmitter 210 and the detecting member 300. The power-supplying member 400 is electrically connected to the transmitter 210.

In the present embodiment, the transmitter 210 and the base 100 are configured as separate structures. Thus, compared with an integrated structure, after use of the device of the present application, the non-reusable base 100 can be discarded, and the transmitter 210 can be reused for another base 100, thereby avoiding waste of components.

The power-supplying member 400 is disposed in, i.e., enclosed with, the base 100. Thus, since the transmitter 210 may need to be reused, compared with the existing technology in which the battery is mounted in the transmitter 210, the present application can avoid the adverse effect, caused by the complicated battery replacement process or repeated charging of the battery, on the service life of the transmitter 210, thereby significantly improving the service life and stability of the transmitter 210.

The detecting terminal 310 extends through and protrudes from the base 100, which allows the detecting terminal 310 to more directly and efficiently come into contact with the patient's skin, greatly enhancing the sensitivity and accuracy of detection. Furthermore, the electrical connection of the base 100 with the transmitter 210 and the detecting member 300 ensures stable and efficient signal transmission.

To address the issue of poor electrical connection stability between the base 100 and the transmitter 210 due to the complicated assembly in related art, in the present embodiment, as shown in FIG. 1 and FIG. 2, the transmitter assembly 200 defines an auxiliary mounting hole 230 extending through the thickness thereof. When the transmitter assembly 200 is mounted on the base 100, the auxiliary mounting hole 230 faces the detecting member 300. The auxiliary mounting hole 230 allows the auxiliary device to pass therethrough, such that the auxiliary device is connected to the detecting member 300.

The shape of the auxiliary mounting hole 230 is not restricted and for instance, can be circular, square, or other shapes. In the embodiment, the shape of the auxiliary mounting hole 230 is not limited, and specifically, the shape can depend on the structure of the auxiliary device.

The auxiliary mounting hole 230 can optionally face the detecting member 300, ensuring that the auxiliary device passed through the auxiliary mounting hole 230 can be precisely aligned with the detecting member 300. Compared with the conventional approach that requires repeated adjustments for alignment and connection, this design can significantly save assembly time and effort, and improve operational convenience.

In this way, with the design of the auxiliary mounting hole 230, after the base 100 is assembled with the transmitter 210, the auxiliary device can be directly inserted through the auxiliary mounting hole 230 into the base 100 and connected to the detecting member 300 in the base 100. Thus, there is no need to firstly mount the sensor on the skin through the auxiliary device, and then mount the transmitter onto the sensor. In the present application, in addition to the above-described assembly approach, it is possible to firstly assemble the base 100 with the transmitter 210 and then mount the auxiliary device. The physiological information monitoring device of the present application supports multiple assembly approached, improving the usage flexibility of the device and enhancing user experience.

Meanwhile, the design of the auxiliary mounting hole 230 enables the physiological information monitoring device to match with various types of auxiliary devices, thereby improving the device versatility and satisfying diverse needs of different scenarios and user groups for physiological information monitoring. For example, in clinical diagnostics, different conditions require specific auxiliary devices; this design allows the monitoring device to be quickly adapted with different auxiliary devices without the need for a complex customized dedicated device.

Additionally, the auxiliary mounting hole 230 extends through the thickness of the transmitter assembly 200, providing stable support and reliable connection for the auxiliary device. Once the auxiliary device extends through the auxiliary mounting hole 230, the auxiliary device is securely connected to the detecting member 300, reducing detection errors or data instability caused by shaking or displacement thereof, thereby ensuring connection reliability in the detection process and improving the accuracy and repeatability of the monitoring data.

Therefore, the physiological information monitoring device provided in the embodiments of the present application simplifies the assembly between the monitoring device and the auxiliary device, saves assembly time, thereby saving time for monitoring while ensuring the assembly stability between the base and the transmitter. Moreover, the interference with the electrical connection between the base and the transmitter caused by the mounting or removal of the auxiliary device is reduced, thereby maximally guaranteeing the stability of the electrical connection between the base and the transmitter.

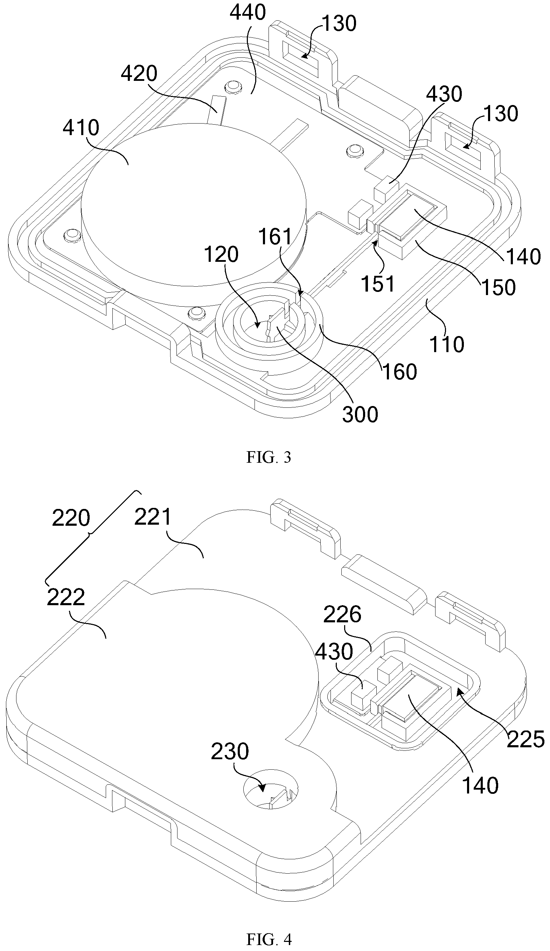

In some embodiments, as shown in FIG. 2 and FIG. 3, the base 100 includes a base seat 110. The base seat 110 has an open structure at a side adjacent to the transmitter assembly 200. For example, the base seat 110 includes a bottom and sidewalls, forming the open structure surrounding an inner space to enclose the power-supplying member 400 and the detecting member 300. The base seat 110 defines a through hole 120. The detecting terminal 310 extends through the through hole 120 and protrudes from the base 100. The auxiliary mounting hole 230 is coaxial with and in communication with the through hole 120.

The auxiliary mounting hole 230 is coaxial with and in communication with the through hole 120, which provides a continuous and stable support structure for the detecting terminal 310. Furthermore, the coaxial and intercommunicated hole structure allows the detecting terminal 310 to distribute stress more evenly when subjected to external forces, preventing deformation or damage to the detecting terminal 310 due to uneven force distribution, thereby enhancing the structural stability of the entire physiological information monitoring device and extending the service life of the device.

Moreover, the connection between the auxiliary device and the detecting terminal 310 can be relatively precise and smooth, effectively reducing detection errors caused by connection misalignment. In addition, the connection process can be simplified, eliminating the need for complex alignment operations by operators during mounting of the auxiliary device, thus reducing mounting difficulty and improving mounting efficiency.

The shape and size of the through hole 120 are not limited, as long as they can be matched with the shape and size of the auxiliary mounting hole 230.

In some embodiments, the transmitter assembly 200 includes a transmitter 210. The transmitter 210 is disposed on the base 100 and covers at least part of the open structure. The auxiliary mounting hole 230 is located in the transmitter 210.

In this way, on the one hand, multiple critical components are reasonably arranged within a limited space, effectively reducing the overall volume of the physiological information monitoring device and making it more compact and portable. On the other hand, the transmitter 210 covers the open structure, providing physical protection for the internal structure in the base seat 110 to a certain extent, preventing damage from external collisions, friction, dust, etc., and improving the stability and reliability of the base 100.

By forming the auxiliary mounting hole 230 in the transmitter 210, the transmitter 210 simultaneously performs the functions of covering the open structure and providing the auxiliary mounting hole 230, which eliminates the requirement for additional structural components, simplifying the structure of the transmitter assembly 200, reducing the manufacturing difficulty and cost, and improving the overall stability of the device.

In some embodiments, as shown in FIG. 2, the transmitter assembly 200 can include a transmitter 210 and a covering member 220. The covering member 220 is disposed on the base 100 and covers at least part of the open structure. The transmitter 210 is disposed on a side of the covering member 220 away from the power-supplying member 400. The auxiliary mounting hole 230 is located in the covering member 220. The transmitter 210 is positioned to avoid the auxiliary mounting hole 230.

The position of the transmitter 210 is not limited. Any position which can avoid the auxiliary mounting hole 230 falls within the protection scope of the present application.

In this way, by disposing the auxiliary mounting hole 230 in the covering member 220, there is no need to create a hole in the transmitter 210, which can improve the structural integrity of the transmitter 210 and is therefore beneficial for extending the service life of the transmitter 210.

The transmitter 210 is positioned to avoid the auxiliary mounting hole 230, which effectively prevents interference between the auxiliary device and the transmitter 210 during connection or use, ensuring smooth connection between the auxiliary device and the detecting member 300, as well as normal operation of the transmitter 210 without mutual influence. This improves the reliability and stability of the entire monitoring system and ensures the accuracy of the monitoring data.

In some embodiments, as shown in FIG. 2 and FIGS. 4 to 8, the covering member 220 can include a covering portion 221 and a connecting portion 222. The covering portion 221 covers at least part of the open structure. The connecting portion 222 and the transmitter 210 are located on the same side of the covering portion 221. The transmitter 210 horizontally abuts against the connecting portion 222. The auxiliary mounting hole 230 extends through both the connecting portion 222 and the covering portion 221. The transmitter 210 is configured to extend through the covering portion 221 and is electrically connected to the base 100.

The assembly relationship between the covering portion 221 and the connecting portion 222 is not limited. For example, the covering portion 221 and the connecting portion 222 can be connected through screws or snap buckles, or the covering portion 221 and the connecting portion 222 can be integrally formed, which is not limited in the embodiments.

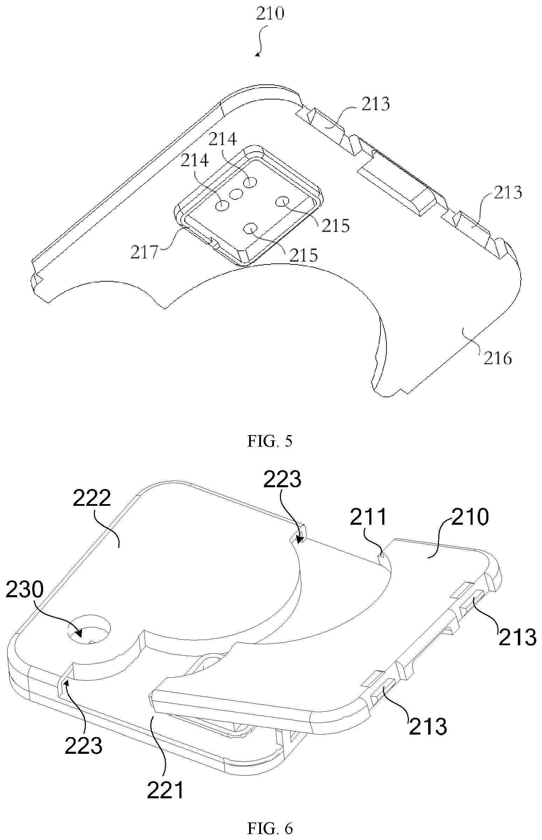

As shown in FIGS. 6 to 8, the transmitter 210 horizontally abuts against the connecting portion 222, which enables relatively uniform and appropriate force transmission. When subjected to the external force caused by such as accidental dropping or collision, the overall structure can better distribute the impact force, avoiding damage due to excessive localized stress, thereby further enhancing the durability and reliability of the device.

In some embodiments, at a position where the connecting portion 222 abuts against the transmitter 210, one of the connecting portion 222 and the transmitter 210 can include a snap buckle 211, while the other of the connecting portion 222 and the transmitter 210 can include a snap groove 223, and the snap buckle 211 is snap-fitted into the snap groove 223.

In these embodiments, as shown in FIG. 6, the transmitter 210 including the snap buckle 211 and the connecting portion 222 including the snap groove 223 are taken as an example for description. In this way, the engagement between the snap buckle 211 and the snap groove 223 can provide a strong force for the mechanical connection, thereby improving the connection stability between the transmitter 210 and the covering member 220.

For example, when the transmitter 210 is connected to the covering portion 221, one of the transmitter 210 and the covering portion 221 can include a sliding block, and the other can include a sliding groove, and the sliding block is capable of sliding in the slide groove. In this way, the position of the transmitter 210 can be adjusted by sliding the sliding block in the sliding groove, thereby improving wearing comfortableness and monitoring effectiveness, which is not limited in the embodiments.

In some other embodiments, an end of the connecting portion 222 can be rotatably connected to an end of the transmitter 210 via a rotating shaft, and the other end of the transmitter 210 can be rotatable around the rotating shaft relative to the connecting portion 222. This configuration provides greater functional flexibility to the device, allowing it to meet signal transmission or reception requirements in different directions and to adapt to various complex monitoring environments.

In some embodiments, at a position where the connecting portion 222 abuts against the transmitter 210, one of the connecting portion 222 and the transmitter 210 can have a convex surface 224, and the other of the connecting portion 222 and the transmitter 210 can have a concave surface 212.

In these embodiments, as shown in FIG. 2, the connecting portion 222 having the convex surface 224 and the transmitter 210 having the concave surface 212 are taken as an example for description. In this way, the cooperation between the convex surface 224 and the concave surface 212 provides spontaneous positioning and guiding for the connecting portion 222 and the transmitter 210. During the abutting process, the convex surface 224 can fit into the concave surface 212, guiding the two members to be in alignment with each other quickly and precisely, thereby reducing errors from manual alignment.

Moreover, the convex surface 224 and the concave surface 212 fit with each other, increasing the contact area and friction force at the connection position. This tightly fitting structure can effectively resist external vibrations, shaking, and minor impacts, preventing relative displacement between the connecting portion 222 and the transmitter 210 during daily use, thereby maintaining the stability of the device.

In some embodiments, one of the base seat 110 and the transmitter 210 can have an inserting protrusion 213, and the other of the base seat 110 and the transmitter 210 can have a fixing buckle 130. The inserting protrusion 213 is correspondingly assembled into the fixing buckle 130.

The shapes, numbers, etc., of the inserting protrusion 213 and the fixing buckle 130 are not limited and can be specifically set according to actual needs. For example, the inserting protrusion 213 can be made of an elastic material. In this way, when the elastic inserting protrusion 213 is assembled into the fixing buckle 130, it can self-adapt to minor dimensional deviations through its own elasticity. Even if there are certain manufacturing tolerances between the base seat 110 and the transmitter 210 during production, the elastic inserting protrusion 213 can fill the gap through deformation, achieving a tight fit, ensuring a secure and reliable connection every time, and thus improving the overall stability of the product quality.

In these embodiments, as shown in FIGS. 6 to 8, the transmitter 210 having the inserting protrusion 213 and the base seat 110 having the fixing buckle 130 is taken as an example for description. This configuration contributes to improving the connection stability between the base seat 110 and the transmitter 210, ensuring the structural strength of the device.

It should be noted that during assembly, the transmitter 210 is tilted so that the snap buckle 211 can be inserted into the snap groove 223, and then the transmitter 210 is pressed downward to snap the inserting protrusion 213 into the fixing buckle 130, thereby completing the mounting and fixation of the transmitter 210. Upon reaching the lifespan, the transmitter 210 can be removed by twisting and prying the fixing buckle 130 outward, for example manually or with a tool, so that the fixing buckle 130 deforms to release the inserting protrusion 213, thereby completing the removal of the transmitter 210.

It should be noted that, in these embodiments, as shown in FIG. 1, the transmitter 210 can include a recessed structure 180 disposed on the side adjacent to the inserting protrusion 213. The recessed structure 180 is configured to facilitate prying open the fixing buckle 130. For example, when the fixing buckle 130 is located at the transmitter 210, as shown in FIG. 1, the recessed structure 180 corresponds in position to the fixing buckle 130. The operator can pry open the fixing buckle 130 via the recessed structure 180, causing the fixing buckle 130 to twist outward relative to the inserting protrusion 213, thereby disengaging the inserting protrusion 213 from the fixing buckle 130 and causing the fixing buckle 130 to release the inserting protrusion 213.

Alternatively, the base seat 110 can include a similar recessed structure. Thus, when the fixing buckle 130 is located at the base seat 110, the corresponding recessed structure on the base seat 110 can likewise facilitates the twist of the fixing buckle 130.

The transmitter 210 can be reused, while the removed sensor can be discarded. The transmitter 210 can be mounted to a new sensor by repeating the mounting steps for the transmitter 210. The outward twisting of the fixing buckle 130 can cause non-destructive elastic deformation thereto.

In some embodiments, as shown in FIGS. 2, 3, and 9, the base seat 110 can be provided with a first conductive member 140. As shown in FIG. 5, the transmitter 210 includes a housing 216, a circuit board, and a second conductive member 214. The circuit board and the second conductive member 214 are disposed in the housing 216. The second conductive member 214 protrudes from the side of the housing 216 facing the base 100.

For example, the first conductive member 140 can be a flexible conductor, such as conductive rubber, e.g., a conductive zebra strip, and the second conductive member 214 can be a conductive pillar. The embodiment is not limited thereto.

For example, the circuit board and the housing 216 can be integrally molded, or can be separated and fitted components that can be assembled together to achieve waterproof and dustproof effects, thereby protecting the electronic components in the housing 216.

The first conductive member 140 is respectively electrically connected to the detecting member 300 and the second conductive member 214, and the second conductive member 214 is electrically connected to the circuit board. This establishes a stable electrical connection path, ensuring that physiological information signals acquired from the detecting member 300 can be reliably transmitted to the circuit board in the transmitter 210 for subsequent processing and transmission.

In addition, during the assembling process, the second conductive member 214 protruding from the housing 216 can be easily docked with the first conductive member 140, simplifying the assembling process. Production personnel can achieve an accurate connection between the two conductive members without complex wiring or alignment procedures, thereby improving production efficiency.

In some embodiments, as shown in FIG. 2 and FIG. 3, the base seat 110 includes a mounting structure defining a mounting groove 150, and the first conductive member 140 is disposed in the mounting groove 150. The mounting structure can have a mounting notch 151, and at least a part of the detecting member 300 extends into the mounting groove 150 via the mounting notch 151.

The formation method, shape, size, etc. of the mounting groove 150 are not limited. For instance, the mounting groove 150 can be a square groove, a circular groove, or an irregularly shaped groove. The mounting groove 150 and the base seat 110 can be integrally formed.

Thus, the design of the mounting groove 150 provides a precise positioning space for the first conductive member 140 and can provide physical protection for the first conductive member 140, thereby ensuring the stability of the electrical connection. The design of the mounting notch 151 allows the detecting member 300 to be smoothly connected to the first conductive member 140, and also provides a certain degree of flexibility for wiring the detecting member 300. Depending on the shape, size and actual assembly requirements of the detecting member 300, the detecting member 300 can appropriately pass through the mounting notch 151 into the mounting groove 150, optimizing the internal space layout.

In addition, the first conductive member 140 and the detecting member 300 can be compressed in the mounting groove 150, so that the conductive rubber of the first conductive member 140 is connected to the conductive area of the detecting member 300. The flexible conductive rubber can compensate for dimensional tolerances between the detecting member 300 and the mounting groove 150, ensuring stable contact therebetween.

In some embodiments, as shown in FIG. 2 and FIG. 3, the base seat 110 can include a limiting structure defining a limiting groove 160. The limiting groove surrounds the opening of the through hole 120. A portion of the detecting member 300 is surrounded by the limiting groove 160. The limiting structure can define a limiting notch 161. The limiting notch 161 is opposite to the mounting notch 151. At least part of the detecting member 300 extends from the mounting notch 151 to the mounting groove 151.

The limiting groove 160 is disposed around the opening of the through hole 120, thereby limiting the position for mounting of the detecting member 300 and effectively restricting movement of the detecting member 300 in all directions, enhancing the stability of the detecting member 300 in the base seat 110.

The limiting notch 161 is opposite to the mounting notch 151, so that during assembly, the detecting member 300 can be quickly and accurately deployed at the designated position through the limiting notch 161 and the mounting notch 151, so as to be connected to the first conductive member 140. This approach reduces complexity of operations or positioning, improves production and assembly efficiency, and reduces labor costs. Meanwhile, the limiting notch 161 being opposite to the mounting notch 151 provides a clear path for wiring the detecting member 300. The orderly wiring not only reduces mess and entanglement of wiring, but also prevents damage to the detecting member 300 and conductive members caused by improper wiring, while facilitating later maintenance and inspection.

In some embodiments, as shown in FIG. 2 and FIG. 5, the power-supplying member 400 can include a third conductive member 430. The transmitter 210 can include a fourth conductive member 215. The fourth conductive member 215 can be disposed in the housing 216 and protrude from the side of the housing 216 facing the base 100. The third conductive member 430 is electrically connected to the fourth conductive member 215.

For example, the third conductive member 430 can be conductive foam, and the fourth conductive member 215 can be a conductive pillar.

The third conductive member 430 is located on a side adjacent to the mounting groove 150. In this design, since the region where the mounting groove 150 is located is the position where signal transmission-related components are located, for example, a region where the first conductive member 140 and the detecting member 300 are connected, disposing the third conductive member 430 of the power-supplying member 400 in this region shortens the length of the power supply line, and reduces mutual interference between the power transmission line and the signal transmission line, thereby improving the electromagnetic compatibility of the entire device and ensuring the accuracy of physiological information monitoring data.

In some embodiments, as shown in FIG. 2 and FIG. 3, the power-supplying member 400 includes a battery 410 and an electrode plate 420 connected to the battery 410. The electrode plate 420 is electrically connected to the third conductive member 430.

For example, the electrode plate 420 includes a positive electrode plate and a negative electrode plate, the positive electrode plate is configured to be connected to the positive electrode of the battery 410, and the negative electrode plate is configured to be connected to the negative electrode of the battery 410.

The electrode plate 420 serves as a connecting bridge between the battery 410 and the third conductive member 430, ensuring that the electrical energy generated by the battery 410 can be efficiently and stably transmitted to the third conductive member 430, thereby supplying power to the transmitter 210 and the entire device. This connection method reduces problems such as poor contact or excessive resistance that may occur in intermediate links, thereby guaranteeing the reliability of the power supply and enabling continuous and stable operation of the device.

In some embodiments, as shown in FIG. 2 and FIG. 3, the power-supplying member 400 can include a substrate 440, the substrate 440 is located in the base seat 110, and the battery 410 and the third conductive member 430 are both disposed on the substrate 440.

For example, the battery 410 and the third conductive member 430 can be fixed to the substrate 440 by soldering. The substrate 440 can be provided with a conductive circuit for electrically connecting the battery 410 and the third conductive member 430. For example, the substrate 440 can be a flexible printed circuit (FPC), a rigid board, or a three-dimensional circuit based on a plastic carrier. Alternatively, the electrode plate 420 can directly extend to the bottom of the third conductive member 430, which is not limited in the embodiments. Any configuration that achieves electrical connection between the battery 410 and the third conductive member 430 falls within the protection scope of the present application.

Thus, during production, the battery 410 and the third conductive member 430 are mounted on the substrate 440 in advance to form a relatively independent power-supplying module, which simplifies the mounting steps in the base. The production personnel only need to assemble the entire substrate 440 into the base and perform a few connection works to complete the assembly of the power supply system, thereby improving production efficiency and reducing production costs.

In addition, by providing the substrate 440, the battery 410 and the third conductive member 430 can be appropriately arranged on the substrate 440. For example, the third conductive member 430 can be positioned on a side adjacent to the transmitter 210 that requires power supply, further shortening the power transmission distance while avoiding interference with other components, optimizing the internal structural layout of the entire device, and improving overall performance.

In some embodiments, as shown in FIG. 2, the covering member 220 can define an opening 225. When the covering member 220 is mounted on the base 100, the first conductive member 140 and the third conductive member 430 are exposed through the opening 225.

In this way, the design of the opening 225 facilitates reliable contact and connection between the first conductive member 140 and the second conductive member 214, and between the third conductive member 430 and the fourth conductive member 215. Furthermore, since the conductive members generate heat during operation, the opening 225 provides an additional path for heat dissipation. Compared with a fully enclosed structure, the opening 225 allows hot air around the conductive members to exchange with external cold air, accelerating heat dissipation, reducing the temperature of the conductive members and surrounding components, benefiting to maintaining good performance of the conductive members and extending their service life.

In some embodiments, as shown in FIG. 2, the covering member 220 can include a first sealing member 226 which can be disposed around the periphery of the opening 225. The transmitter 210 can include a second sealing member 217 which can be disposed on the side adjacent to the base 100. The second sealing member 217 surrounds the second conductive member 214 and the fourth conductive member 215.

For example, the first sealing member 226 and the second sealing member 217 can be sealing rings, which can be made of silicone or rubber, for waterproof sealing.

In this way, the first sealing member 226 around the opening 225 can effectively prevent dust, moisture, impurities and other external substances from entering the relevant devices, benefiting to maintaining a specific internal environment of the device, such as maintaining certain temperature, humidity, or pressure conditions.

The second sealing member 217 can effectively prevent accidental electrical contact between the conductive members and other objects in the surrounding environment, avoiding short-circuit situations.

In some embodiments, one of the first conductive member 140 and the second conductive member 214 can be a rigid member, and the other of the first conductive member 140 and the second conductive member 214 can be a flexible member. One of the third conductive member 430 and the fourth conductive member 215 can be a rigid member, and the other of the third conductive member 430 and the fourth conductive member 215 can be a flexible member.

For example, the first conductive member 140 and the third conductive member 430 can be elastic flexible conductors. The second conductive member 214 and the fourth conductive member 215 can be rigid conductive pillars, such as copper pillars.

In this way, the rigid conductive members have fixed shapes and structures, providing stable support and positioning for the electrical connection, while the flexible conductive member can be bent and twisted arbitrarily, easily adapting to various complex spatial layouts during assembly. Furthermore, the rigid members are easy to be mounted and fixed in specific positions, while the flexible members can be connected to the rigid members through simple bending or adjustment, without complex machining processing or precise positioning operations, providing redundancy by compression of the flexible connection to compensate for poor contact caused by component tolerances and fitting gaps, making the connection between components more stable and reliable.

In some embodiments, as shown in FIG. 1, the base 100 can define an auxiliary assembly groove 170 on a side thereof, the auxiliary assembly groove 170 is configured for fixing the base 100 to the additional auxiliary device to facilitate wearing.

The physiological information monitoring device provided in the embodiments of the present application simplifies the assembly between the monitoring device and the auxiliary device, saves operation time, and ensures the assembly stability between the base and the transmitter. Moreover, the interference with the electrical connection between the base and the transmitter caused by the mounting or removal of the auxiliary device is reduced, thereby maximally guaranteeing the stability of the electrical connection between the base and the transmitter.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A physiological information monitoring device configured to cooperate with an auxiliary device, the physiological information monitoring device comprising a base (100), a transmitter assembly (200), a detecting member (300), and a power-supplying member (400), the transmitter assembly (200) at least comprising a transmitter (210);
wherein the transmitter assembly (200) is disposed on the base (100), the power-supplying member (400) and the detecting member (300) are disposed in the base (100), the detecting member (300) has a detecting terminal (310), and the detecting terminal (310) extends through and protrudes from the base (100); the base (100) is electrically connected to the transmitter (210) and the detecting member (300), respectively, and the power-supplying member (400) is electrically connected to the transmitter (210);
wherein the transmitter assembly (200) defines an auxiliary mounting hole (230) extending through a thickness of the transmitter assembly (200); when the transmitter assembly (200) is mounted on the base (100), the auxiliary mounting hole (230) faces the detecting member (300), and the auxiliary mounting hole (230) allows the auxiliary device to extend therethrough, such that the auxiliary device is connected to the detecting member (300).

2. The physiological information monitoring device according to claim 1, wherein the base (100) comprises a base seat (110), the base seat (110) has an open structure at a side adjacent to the transmitter assembly (200), the base seat (110) defines a through hole (120), and the detecting terminal (310) extends through the through hole (120);
the auxiliary mounting hole (230) and the through hole (120) are coaxial and in communication with each other.

3. The physiological information monitoring device according to claim 2, wherein the transmitter assembly (200) comprises a transmitter (210), the transmitter (210) is disposed on the base (100) and covers at least part of the open structure, and the auxiliary mounting hole (230) is located in the transmitter (210).

4. The physiological information monitoring device according to claim 2, wherein the transmitter assembly (200) comprises a transmitter (210) and a covering member (220), the covering member (220) is disposed on the base (100) and covers at least part of the open structure;
the transmitter (210) is disposed on a side of the covering member (220) away from the power-supplying member (400), the auxiliary mounting hole (230) is located in the covering member (220), and the transmitter (210) is disposed beside the auxiliary mounting hole (230).

5. The physiological information monitoring device according to claim 4, wherein the covering member (220) comprises a covering portion (221) and a connecting portion (222), the covering portion (221) covers at least part of the open structure;
the connecting portion (222) and the transmitter (210) are located on a same side of the covering portion (221), the transmitter (210) horizontally abuts against the connecting portion (222), the auxiliary mounting hole (230) extends through both the connecting portion (222) and the covering portion (221), and the transmitter (210) is configured to extend through the covering portion (221) and is electrically connected to the base (100).

6. The physiological information monitoring device according to claim 5, wherein at a position where the connecting portion (222) abuts against the transmitter (210),
one of the connecting portion (222) and the transmitter (210) comprises a snap buckle (211), and another one of the connecting portion (222) and the transmitter (210) is comprises a snap groove (223), and the snap buckle (211) is snap-fitted into the snap groove (223); or
an end of the connecting portion (222) is rotatably connected to an end of the transmitter (210) via a rotating shaft, and another end of the transmitter (210) is rotatable around the rotating shaft relative to the connecting portion (222).

7. The physiological information monitoring device according to claim 5, wherein at a position where the connecting portion (222) abuts against the transmitter (210), one of the connecting portion (222) and the transmitter (210) has a convex surface (224), and another one of the connecting portion (222) and the transmitter (210) has a concave surface (212).

8. The physiological information monitoring device according to any one of claims 2 to 7, wherein one of the base seat (110) and the transmitter (210) has an inserting protrusion (213), and another one of the base seat (110) and the transmitter (210) has a fixing buckle (130), and the inserting protrusion (213) is assembled into the fixing buckle (130).

9. The physiological information monitoring device according to any one of claims 2 to 8, wherein the base seat (110) is provided with a first conductive member (140), the transmitter (210) comprises a housing (216), a circuit board, and a second conductive member (214), the circuit board and the second conductive member (214) are disposed in the housing (216), the second conductive member (214) protrudes from a side of the housing (216) facing the base (100), the first conductive member (140) is electrically connected to the detecting member (300) and the second conductive member (214), respectively, and the second conductive member (214) is electrically connected to the circuit board.

10. The physiological information monitoring device according to claim 9, wherein the base seat (110) comprises a mounting structure defining a mounting groove (150), the first conductive member (140) is disposed in the mounting groove (150), the mounting structure defines a mounting notch (151), and at least a part of the detecting member (300) extends into the mounting groove (150) via the mounting notch (151).

11. The physiological information monitoring device according to claim 9 or 10, wherein the power-supplying member (400) comprises a third conductive member (430); the transmitter (210) comprises a fourth conductive member (215); the fourth conductive member (215) is disposed in the housing (216) and protrudes from the side of the housing (216) facing the base (100); the third conductive member (430) is electrically connected to the fourth conductive member (215);
the third conductive member (430) is located on a side adjacent to the mounting groove (150).

12. The physiological information monitoring device according to claim 11, wherein the power-supplying member (400) comprises a battery (410) and an electrode plate (420) connected to the battery (410); the electrode plate (420) is electrically connected to the third conductive member (430).

13. The physiological information monitoring device according to claim 12, wherein the power-supplying member (400) further comprises a substrate (440), the substrate (440) is located in the base (110), and the battery (410) and the third conductive member (430) are both disposed on the substrate (440).

14. The physiological information monitoring device according to any one of claims 11 to 13, wherein the covering member (220) defines an opening (225); when the covering member (220) is mounted on the base (100), the first conductive member (140) and the third conductive member (430) are exposed through the opening (225).

15. The physiological information monitoring device according to any one of claims 11 to 14, wherein one of the first conductive member (140) and the second conductive member (214) is a rigid member, and the other of the first conductive member (140) and the second conductive member (214) is a flexible member; and/or
one of the third conductive member (430) and the fourth conductive member (215) is a rigid member, and the other of the third conductive member (430) and the fourth conductive member (215) is a flexible member.
